# FASCICULE DE BREVET EUROPEEN

(11) **EP 2 307 353 B1**
(45) Date de publication et mention de la délivrance du brevet: **29.08.2012**
(21) Numéro de dépôt: 09784500.2
(22) Date de dépôt: 09.07.2009
(51) Int. Cl.: C07C 227/08, C07C 229/08

(54) **PROCEDE DE SYNTHESE DE L'ACIDE AMINO-9-NONANOÏQUE OU DE SES ESTERS A PARTIR D'ACIDES GRAS NATURELS INSATURES**
VERFAHREN ZUR SYNTHESE VON 9-AMINONONANSÄURE ODER DEN ESTERN DAVON AUS NATÜRLICHEN UNGESÄTTIGTEN FETTSÄUREN
METHOD FOR SYNTHESISING 9-AMINONONANOIC ACID OR THE ESTERS THEREOF FROM NATURAL UNSATURATED FATTY ACIDS

(30) Priorité: 10.07.2008 FR 0854708
(43) Date de publication de la demande: 13.04.2011
(73) Titulaire: ARKEMA FRANCE, 92700 Colombes (FR)
(72) Inventeur: DUBOIS, Jean-Luc, F-69390 Millery (FR)
(86) Numéro de dépôt international: PCT/FR2009/051370
(87) Numéro de publication internationale: WO 2010/004220

(56) Documents cités:
- WO-A-2008/046106
- MILLER W R ET AL: "NYLON-9 VIA 9-AMINONONANOIC ACID FROM SOYBEAN OIL" IND. ENG. CHEM. RES, WEB, vol. 10, no. 4, 1 janvier 1971 (1971-01-01), pages 442-447, XP002492150 ISSN: 0536-1079
- KOHLHASE W L ET AL: "9-Aminononanamide and Nylon-9 From Azelaaldehydic Derivatives of Soybean Oil" JOURNAL OF THE AMERICAN OIL CHEMISTS' SOCIETY, SPRINGER, BERLIN, DE, vol. 47, 1 janvier 1970 (1970-01-01), pages 183-188, XP002492148 ISSN: 0003-021X
- MOL J C: "APPLICATION OF OLEFIN METATHESIS IN OLEOCHEMISTRY: AN EXAMPLE OF GREEN CHEMISTRY" GREEN CHEMISTRY, ROYAL SOCIETY OF CHEMISTRY, CAMBRIDGE, GB, vol. 4, 1 janvier 2002 (2002-01-01), pages 5-13, XP008068603 ISSN: 1463-9262

## Description

L'invention vise un procédé de synthèse de l'acide amino-9-nonanoïque ou de ses esters à partir d'acides gras naturels insaturés comportant au moins une étape de métathèse de l'acide gras naturel et une étape d'oxydation par coupure oxydante.

L'industrie des polyamides utilise toute une gamme de monomères constitués par des ω-amino-acides à longue chaîne, usuellement appelés Nylon, caractérisés par la longueur de chaîne méthylène (-CH₂)ₙ séparant deux fonctions amide -CO-NH-, C'est ainsi que sont connus les Nylon-6, Nylon 6-6, Nylon 6-10, Nylon 7, Nylon 8, Nylon 9, Nylon 11, Nylon 13, etc...

Ces monomères sont par exemple fabriqués par voie de synthèse chimique en utilisant notamment comme matière première des oléfines en C2 à C4, des cycloalcanes ou du benzène, mais aussi de l'huile de ricin (Nylon 11), de l'huile érucique ou lesquérolique (Nylon 13)...

L'évolution actuelle en matière d'environnement conduit dans les domaines de l'énergie et de la chimie à privilégier l'exploitation des matières premières naturelles provenant d'une source renouvelable. C'est la raison pour laquelle certains travaux ont été repris pour élaborer sur le plan industriel des procédés utilisant des acides/esters gras comme matière première de fabrication de ces monomères.

Ce type d'approche n'a que peu d'exemples industriels. Un des rares exemples de procédé industriel utilisant un acide gras comme matière première est celui de la fabrication à partir de l'acide ricinoléique extrait de l'huile de ricin, de l'acide amino-11-undécanoique, qui est à la base de la synthèse du Rilsan 11 ^{®}. Ce procédé est décrit dans l'ouvrage « Les Procédés de Pétrochimie » de A. Chauvel et al. paru aux Editions TECHNIP (1986). L'acide amino-11-undécanoique est obtenu en plusieurs étapes. La première consiste en une méthanolyse de l'huile de ricin en milieu basique produisant le ricinoléate de méthyle qui est ensuite soumis à une pyrolyse pour obtenir, d'une part l'heptanaldéhyde et, d'autre part le undécylénate de méthyle. Ce dernier est passé en forme acide par hydrolyse. Ensuite l'acide formé est soumis à une hydrobromuration pour donner l'acide ω-bromé d'où l'on passe par amination à l'acide amino-11-undécanoïque.

Le procédé de l'invention vise la synthèse de l'acide 9-amino-nonanoïque ou acide 9-aminoazélaïque correspondant au Nylon 9. En ce qui concerne ce monomère particulier on peut citer *l'ouvrage* «n-Nylons, Their Synthesis, Structure and Properties » - 1997 Ed.J. Wiley et Sons dont le chapitre 2.9 (pages 381 à 389) est consacré au 9-Nylon. Cet article donne la synthèse des réalisations et des travaux conduits sur le sujet. On y mentionne, page 381, le procédé développé par l'ex Union Soviétique ayant conduit à la commercialisation du Pelargon®. On y mentionne, page 384, également un procédé développé au Japon utilisant l'acide oléique venant l'huile de soja comme matière première. La description correspondante fait référence à l'ouvrage de A. Ravve « Organic Chemistry of Macromolecules » (1967) Marcel Dekker, Inc., dont la partie 15 est consacrée aux polyamides et qui mentionne en page 279 l'existence d'un tel procédé.

Pour être complet sur l'état de l'art en la matière, il faut citer les nombreux articles publiés par E. H. Pryde et al. entre 1962 et 1975 dans - Journal of the American Oil Chemists ' Society - « Aldehydic Materials by the Ozonization of Vegetable Oils » Vol. 39 pages 496-500 ; « Pilot Run, Plant Design and Cost Analysis for Reductive Ozonolysis of Methyl Soyate » Vol. 49 pages 643-648 et R.B. Perkins et al. « Nylon-9 from Unsaturated Fatty Derivatives : Preparation and Characterization » JAOCS, Vol. 52 pages 473-477. Il est à noter que le premier de ces articles fait aussi référence page 498 à des travaux antérieurs réalisés par des Japonais : H. Otsuki et H. Funahashi.

On peut également citer l'article de Miller W R et al. : « Nylon-9 via 9-aminononanoic acid from soybean oil » IND. ENG. CHEM. RES., WEB, Vol. 10, no. 4, 1 er janvier 1971, pages 442-447, et l'article de Kohlhase W L et al : "9-aminononanamide and Nylon-9 from azelaaldehydic derivatives of soybean oil" Journal of the American oil chemists society, Springer, Berlin, DE, vol. 47 1er janvier 1970, pages 183-188.

Pour résumer cet état de l'art visant la synthèse du « Nylon 9 » à partir d'huiles végétales on peut décrire le mécanisme réactionnel simplifié suivant appliqué à l'ester oléique, extrait des huiles par méthanolyse :

### Ozonolyse réductrice

H₃C-(CH₂)₇-CH=CH-(CH₂)₇-COOCH₃ + (O₃, H₂) → HOC-(CH₂)₇-COOCH₃ + H₃C-(CH₂)₇-COH

### Amination réductrice

HOC-(CH₂)₇-COOCH₃ + (NH₃, H₂) → H₂N-(CH₂)₈-COOCH₃ + H₂O

### Hydrolyse

H₂NC-(CH₂)₈-COOCH₃ +H₂O → H₂N-(CH₂)₈-COOH + CH₃OH

Cette voie très séduisante sur le plan réactionnel présente cependant un inconvénient économique important constitué par la production lors de la première étape d'un aldéhyde à longue chaîne (9 atomes de carbone au total) pratiquement pas valorisable notamment dans l'industrie des polymères de type polyamides.

La présente invention vise à pallier cet inconvénient majeur en proposant un procédé, fondé sur un processus réactionnel voisin, mais permettant la synthèse d'une gamme de composés pouvant être valorisés aisément.

Le problème est donc de trouver un procédé de synthèse d'un ω-aminoacide de formule H₂N-(CH₂)₈-COOH (et de son polymère) à partir de matières premières renouvelables de très large accès, et donc peu onéreuses, simple à mettre en oeuvre tout en évitant d'une part les contraintes environnementales évoquées précédemment et d'autre part les handicaps économiques dus aux sous-produits des réactions.

La solution proposée consiste à travailler à partir de matières premières constituées par des acides gras insaturés à longue chaîne naturels. On entend par acide gras naturel un acide issu des milieux végétal ou animal, y compris les algues, plus généralement du règne végétal, et donc renouvelable. Cet acide comportera au moins une insaturation oléfinique localisée en position 9 par rapport au groupement acide (delta 9) et comportant au moins 10 et de préférence au moins 14 atomes de carbone par molécule.

On peut citer à titre d'exemples de tels acides, l'acide caproléique (9-décénoïque), l'acide myristoléique (cis-9-tétradécénoïque), l'acide palmitoléique (cis-9-hexadécénoïque), les acides en C18, oléique (cis-9-octadécénoïque), élaidique (trans-9-octadécénoïque), linoléiques (cis,cis-9,12-octadécadiènoïque et cis,trans-9,11-octadécadiènoïque), α-linolénique (cis,cis,cis-9,12,15-octadécatriènoïque), α-éléostéarique (cis,trans,trans-9,11,13-octadécatriènoïque, l'acide gras hydroxylé ricinoléique (12-hydroxy--cis-9-octadécénoïque), et l'acide gadoléique (cis-9-eicosénoïque).

Ces divers acides sont issus des huiles végétales extraites de diverses plantes telles que le tournesol, le colza, le ricin, l'olive, le soja, le palmier, l'avocat, l'argousier.

Ils sont issus également du monde animal terrestre ou marin, et dans ce dernier cas, aussi bien sous forme de poissons, de mammifères que d'algues. Il s'agit alors en général de graisses provenant de ruminants, de poissons comme la morue, ou de mammifères marins comme les baleines ou les dauphins.

L'invention a pour objet un procédé de synthèse d'aminoacides ou d'aminoesters de formule générale NH₂-(CH₂)₈-COOR, R étant soit H, soit un radical alkyle comportant de 1 à 4 atomes de carbone, à partir d'acides ou d'esters gras naturels insaturés à longue chaîne répondant à la formule R₁-CH=CH-(CH₂)₇-COOR dans laquelle R₁ représente soit H, soit un radical hydrocarboné comprenant de 1 à 11 atomes de carbone, comportant de 0 à 2 insaturations oléfiniques et le cas échéant une fonction hydroxyle, consistant, dans une première étape à soumettre ledit acide ou ester gras insaturé à une réaction de métathèse catalytique croisée avec l'éthylène pour former des acides ou esters ω-insaturés dont au moins 50% molaires répondent à la formule CH₂=CH-(CH₂)₇-COOR, puis dans une deuxième étape à soumettre l'acide ou ester de formule CH₂=CH-(CH₂)₇-COOR à une réaction de coupure oxydante pour former un aldéhyde-acide/ester de formule CHO-(CH₂)₇-COOR, puis enfin à transformer le produit résultant par amination réductrice en ω-amino-acide/ester (à moins que l'on ait dit dans le texte que acide doit être compris comme acide ou ester) de formule NH₂-(CH₂)₈-COOR.

Le processus réactionnel peut se résumer comme suit appliqué à l'acide oléique

CH₃-(CH₂)₇-CH=CH-(CH₂)₇-COOH + CH₂=CH₂ ⇔ CH₂=CH-(CH₂)₇-COOH + CH₂=CH-(CH₂)₇-CH₃

CH₂=CH-(CH₂)₇-COOH + (Oxydant, H₂*) → COH- (CH₂)₇-COOH + HCHO

COH- (CH₂)₇-COOH + (NH₃, H₂) → NH₂-(CH₂)₈-COOH + H₂O

H₂^{*} symbolyse dans la réaction 2, le couplage d'une oxydation suivie d'une réduction.

Les seuls « sous-produits » formés sont une α-oléfine à longue chaîne et du formaldéhyde.

La réaction de métathèse croisée avec l'éthylène (éthénolyse) est réalisée dans des conditions parfaitement connues. La température de réaction est comprise entre 20 et 100 °C à la pression atmosphérique en présence d'un catalyseur à base de ruthénium.

Les catalyseurs au ruthénium sont choisis de préférence parmi les catalyseurs chargés ou non-chargés de formule générale :
(X1)a (X2)bRu(carbène C) (L1)c(L2)d
   dans laquelle :
   - a, b, c, d sont des nombres entiers avec a et b égaux à 0,1 ou 2 ; c et d égaux à 0, 1, 2, 3 ou 4 ;
   - X1 et X2, identiques ou différents, représentent chacun un ligand mono- ou multi-chélatant, chargé ou non ; à titre d'exemples, on pourra citer les halogénures, le sulfate, le carbonate, les carboxylates, les alcoolates, les phénates, les amidures, le tosylate, l'hexafluorophosphate, le tétrafluoroborate, le bis-triflylamidure, le tétraphénylborate et dérivés. X1 ou X2 peuvent être liés à Y1 ou Y2 ou au (carbène C) de façon à former un ligand bidenté (ou chélate) sur le ruthénium ; et
   - L1 et L2 identiques ou différents, sont des ligands donneurs d'électrons tels que phosphine, phosphite, phosphonite, phosphinite, arsine, stilbine, une oléfine ou un aromatique, un composé carbonylé, un éther, un alcool, une amine, une pyridine ou dérivé, une imine, un thioéthers, ou un carbène hétérocyclique
L1 ou L2 peuvent être liés au "carbène C " de façon à former un ligand bidenté ou chélate,
Le "carbène C" pourra être représenté par la formule générale : C_(R1)_(R2) pour laquelle R1 et R2 sont identiques ou différents tels que l'hydrogène ou tout autre groupe hydrocarbonyle saturé ou insaturé, cyclique, branché ou linéaire, ou aromatique. A titre d'exemples, on pourra citer les complexes du ruthénium alkylidènes, ou cumulènes tels que les vinylidènes Ru=C=CHR ou allénylidènes Ru=C=C=CR1 R2 ou indénylidènes.

Un groupe fonctionnel permettant d'améliorer la rétention du complexe du ruthénium dans le liquide ionique peut être gréffé sur au moins l'un des ligands X1, X2, L1, L2, ou sur le carbène C. Ce groupe fonctionnel peut être chargé ou non chargé tel que de préférence un ester, un éther, un thiol, un acide, un alcool, une amine, un hétérocycle azoté, un sufonate, un carboxylate, un ammonium quaternaire, un guanidinium, un phosphonium quaternaire, un pyridinium, un imidazolium, un morpholinium ou un sulfonium.

La réaction de coupure oxydante de la double liaison, qui conduit à la formation de fonctions aldéhydes sur les deux carbones de la double liaison, est également en elle-même connue.

Elle peut être réalisée au moyen d'un oxydant fort tel que KMnO₄ sous forme concentrée et à la chaleur ainsi que cela est décrit dans « Organic Chemistry » de L.G. Wade Jr. 5th Edition Chapter 8 Reactions of Alkenes. La coupure oxydante peut être effectuée avec de l'eau oxygénée comme décrit dans le brevet GB 743491. L'article de F. Drawert et al. dans Chem. Mikrobiol. Technol. Lebensm. 1, 158-159 (1972) décrit une voie alternative par irradiation de l'huile de tournesol. D'autre part l'article de G.S. Zhang et al. dans Chinese Chemical Letters, Vol 5, N°2, pp105-108 de 1994, indique qu'il est possible d'effectuer la coupure oxydante à partir du diol correspondant à l'acide oléique (voir Entrée 29 du tableau). Cette coupure oxydante est effectuée en utilisant le Chlorochromate d'ammonium comme oxydant. Le diol quant à lui est obtenu par époxydation de l'acide oléique suivie d'une hydrolyse du pont époxy. Elle peut être réalisée par d'autres oxydants tels que l'eau oxygénée et plus particulièrement l'ozone.

Il faut cependant éviter que la réaction d'oxydation soit complète. En effet l'oxydation d'un acide insaturé est la voie de synthèse bien connue pour la fabrication des diacides. Il y a donc lieu de prévoir des conditions opératoires telles que l'on s'arrête à la fonction aldéhyde. C'est la raison pour laquelle lors des travaux décrits dans l'art antérieur on s'est intéressé à l'hydrogénation et/ou à la réduction des produits de l'oxydation (ozonide), très généralement par ozonolyse réductrice. Les conditions d'oxydation peuvent donc être conduites dans des conditions plus douces, et ainsi mieux maîtriser le processus en travaillant en présence d'hydrogène et/ou d'un agent réducteur doux. C'est cette réaction que l'on dénomme l'ozonolyse réductrice.

La réaction d'ozonolyse a fait l'objet de travaux importants qui ont permis de dégager un mécanisme réactionnel dit de « Criegee » (cf Article « Aldehydic Materials by the Ozonization of Vegetable Oils » Vol. 39 pages 496-500 , cité ci-dessus) marqué par la formation d'un ozonide.

La première phase de l'ozonolyse réductrice peut être réalisée dans différents milieux solvants. Si elle est conduite en phase aqueuse, l'acide gras insaturé est présent sous la forme d'une émulsion eau dans l'huile. Elle peut être conduite dans un solvant de type alcool, méthanol, éthanol, propanol, butanol, méthoxyéthanol, cyclohexanol, alcool benzylique ; dans le cas où l'ozonolyse est réalisée sur l'ester gras, il sera avantageux d'utiliser l'alcool R-OH correspondant à l'ester gras. Il a également été proposé par Chris Schwartz, Joseph Raible, Kyle Mott, et Patrick H. Dussault, Tetrahedron 62 (2006), pp. 10747-10752, d'utiliser le DMSO comme milieu solvant. Au milieu solvant alcool, il est souvent possible d'associer un acide organique, en général, l'acide acétique qui sera présent en général sous forme d'un mélange équimoléculaire avec l'alcool.

La seconde phase de l'ozonolyse réductrice va consister en une réduction de l'ozonide qui peut être réalisée avec du zinc dans l'acide acétique, une hydrogénation en présence d'un catalyseur d'hydrogénation (Pd par exemple) ou à l'aide d'un agent réducteur tel que par exemple le diméthylesulfure (DMS).

La variante préférée de réalisation de cette étape est l'ozonolyse réductrice qui pourra réalisée en présence de zinc métal, sous forme de poudre, ou encore de préférence en présence de diméthylesulfure (DMS : CH₃-S-CH₃); en effet ce DMS sera transformé lors de l'ozonolyse réductrice en DMSO, solvant largement utilisé par l'industrie.

Enfin, l'amination réductrice de la fonction aldéhyde en amine primaire est bien connue de l'homme de l'art. L'amination réductrice de l'acide 9-oxononanoïque obtenu pour former l'acide amino-9-nonanoïque peut être effectuée selon de nombreuses méthodes, catalytiques ou enzymatiques, et par exemple selon la méthode décrite dans la demande de brevet US 5,973,208.

Dans une variante de réalisation du procédé de l'invention on pourra ajouter une étape intermédiaire dans le procédé de l'invention. Le procédé se présenterait alors comme suit (à partir de l'acide ou de l'ester):
1^{ère} étape : éthénolyse de l'acide gras

   R₁-CH=CH-(CH₂)₇-COOH + CH₂=CH₂ ⇔ CH₂=CH-(CH₂)₇-COOH + CH₂=CH-R₁
2^{ème} étape : après séparation de l'oléfine, par exemple par stripping, homométathèse de l'acide 9-décénoïque

   CH₂=CH-(CH₂)₇-COOH + CH₂=CH-(CH₂)₇-COOH ⇔ COOH-(CH₂)₇- CH=CH-(CH₂)₇-COOH + CH₂=CH₂
3^{ème} étape : Coupure oxydante (Ozonolyse réductrice)

   COOH-(CH₂)₇-CH=CH-(CH₂)₇-COOH (Oxydant, H₂^{*}) → 2 COH-(CH₂)₇-COOH + H₂O
4^{ème} étape : Amination réductrice

   2 COH-(CH₂)₇-COOH + (NH₃, H₂) → 2 NH₂-(CH₂)₈-COOH + 2 H₂O

H₂^{*} symbolyse dans la réaction 3, le couplage d'une oxydation suivie d'une réduction.

Dans le procédé de l'invention, on peut traiter l'acide gras soit sous sa forme acide soit sous sa forme ester. Le passage d'une forme à l'autre, par méthanolyse, estérification ou hydrolyse, parfaitement banal ne constitue pas une transformation chimique au sens du procédé.

Ainsi, dans un mode de réalisation de l'invention, au terme de la première étape, on sépare l'α-oléfine du milieu pour soumettre lors d'une deuxième étape l'acide/ester insaturé de formule CH₂=CH-(CH₂)₇-COOR à une homométathèse pour former le diacide(diester) de formule ROOC-(CH₂)₇-CH=CH-(CH₂)₇-COOR, puis, dans une troisième étape on soumet le diacide(diester) COOR-(CH₂)₇-CH=CH-(CH₂)₇-COOR à une réaction de coupure oxydante pour former un aldéhyde-acide de formule CHO-(CH₂)₇-COOR , puis enfin, dans une quatrième étape à transformer le produit résultant par une amination réductrice en ω-amino-acide/ester de formule NH₂-(CH₂)₈-COOR

Tous les mécanismes détaillés ci-dessous illustrent, pour faciliter l'exposé, la synthèse des acides. Cependant, la métathèse est aussi efficace avec un ester et même plus efficace, le milieu étant généralement plus anhydre. De la même manière, les schémas illustrent des réactions avec l'isomère cis des acides (ou esters) ; les mécanismes sont aussi bien applicables aux isomères trans.

Le mécanisme réactionnel de cette réaction est illustrée par le schéma 1 ci-dessous 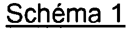 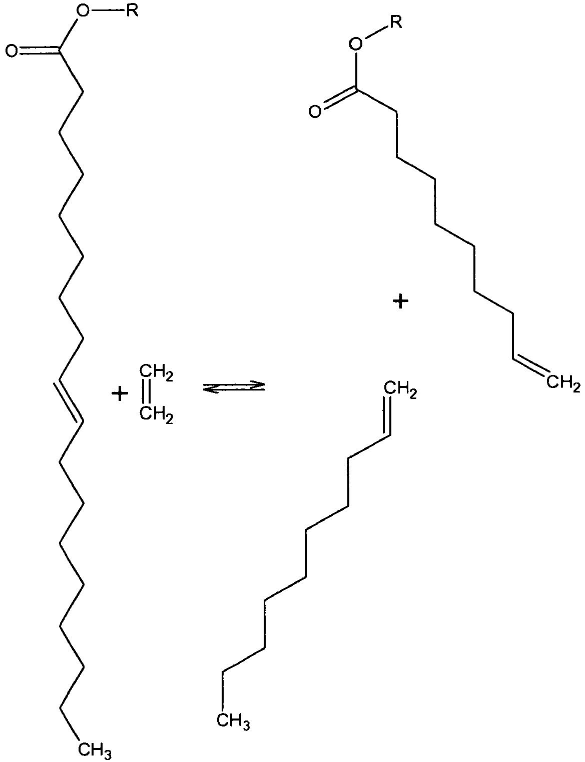 L'homométathèse de l'acide oméga oléfinique est illustrée dans le schéma 2. 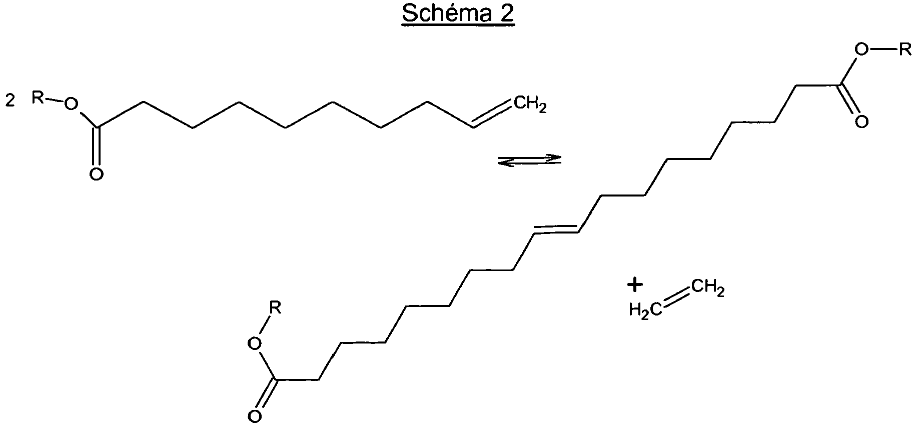 L'ozonolyse réductrice du diacide ou de l'acide ω-nonènoïque est illustrée dans le schéma 3. 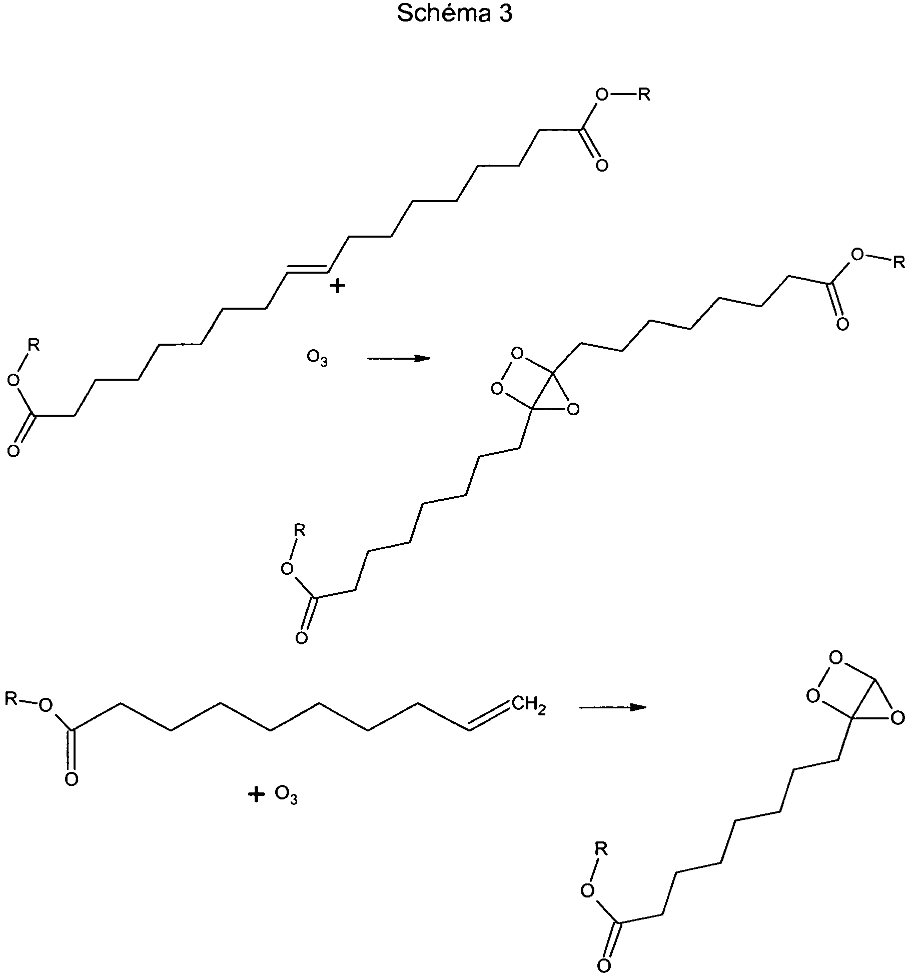 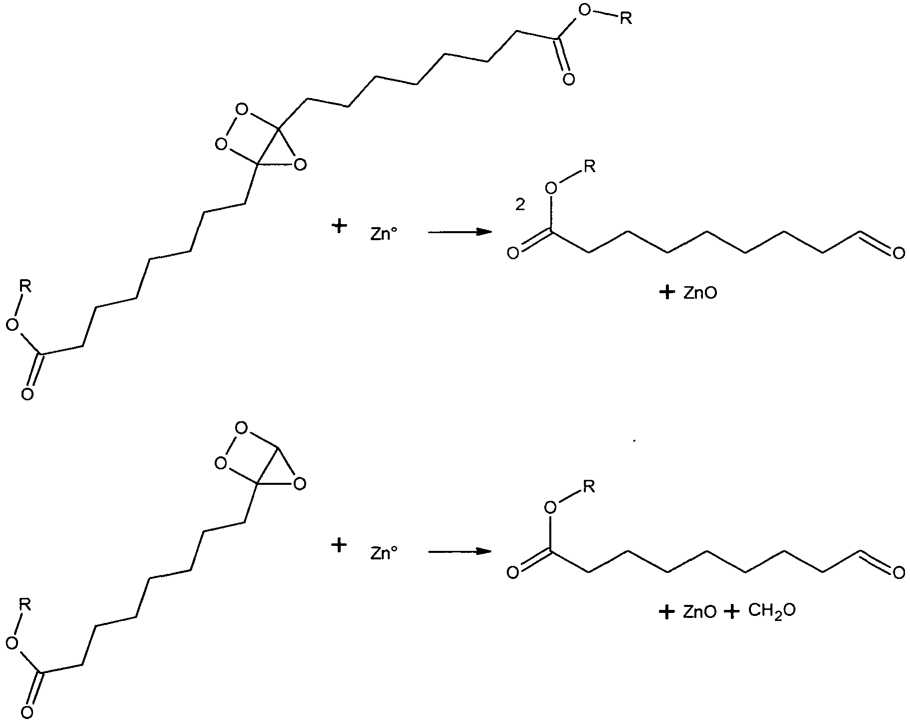 L'amination réductrice est illustrée dans le schéma 4.

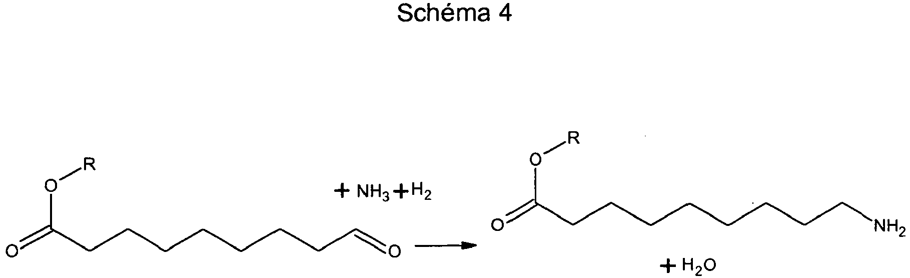

Les réactions de métathèse sont connues depuis longtemps même si leurs applications industrielles sont relativement limitées. On peut se référer à propos de leur utilisation dans la transformation des acides (esters) gras, à l'article de J.C. Mol « Catalytic metathésis of unsaturated fatty acid esters and oil » paru dans Topics in Catalysis Vol. 27, Nos. 1-4, February 2004 (Plenum Publishing Corporation).

La catalyse de la réaction de métathèse a fait l'objet de très nombreux travaux et le développement de systèmes catalytiques sophistiqués. On peut citer par exemple les complexes au tungstène développés par Schrock et al (J. Am. Chem. Soc. 108 (1986) 2771 ou Basset et al. Angew. Chem., Ed. Engl. 31 (1992) 628. Plus récemment, sont apparus les catalyseurs dits de Grubbs (Grubbs et al. Angew. Chem., Ed. Engl. 34 (1995) 2039 et Organic Lett. 1 (1999) 953) qui sont des complexes ruthénium-benzylidène. Il s'agit de catalyse homogène. Il a été aussi développé des catalyseurs hérétogènes à base de métaux tels que Rhénium, Molybdène et Tungstène déposés sur alumine ou silice. Enfin des travaux ont été conduits pour la réalisation de catalyseurs immobilisés, c'est-à-dire de catalyseurs dont le principe actif est celui du catalyseur homogène, notamment les complexes ruthénium-carbène, mais qui est immobilisé sur un support inactif. L'objectif de ces travaux est d'augmenter la sélectivité de la réaction de métathèse croisée vis-à-vis des réactions parasites telles que les « homo-métathèses » entre les réactifs mis en présence. Ils portent non seulement sur la structure des catalyseurs mais également sur l'incidence du milieu réactionnel et les additifs pouvant être introduits.

Dans le procédé de l'invention, tout catalyseur de métathèse actif et sélectif pourra être utilisé. On utilisera cependant de préférence des catalyseurs à base de ruthénium.

La réaction de métathèse croisée de la première étape est conduite à une température comprise entre 20 et 100°C à une pression de 1 à 30 bars en présence d'un catalyseur de métathèse classique par exemple de type Ruthénium. Le temps de réaction est choisi en fonction des réactifs mis en oeuvre et pour atteindre au plus près l'équilibre de la réaction. La réaction est effectuée sous une pression d'éthylène.

La réaction d'homométathèse de la deuxième étape est conduite à une température comprise entre 20 et 100°C à pression inférieure à 5 bars permettant de soutirer l'éthylène en continu et cela avec un catalyseur Ruthénium de type de ceux décrits ci-dessus.

L'invention concerne en outre l'aminoacide ou aminoester d'origine renouvelable de formule générale NH₂-(CH₂)₈-COOR, R étant soit H, soit un radical alkyle comportant de 1 à 4 atomes de carbone.

Par aminoacides ou aminoesters d'origine renouvelable, on entend les aminoacides ou aminoesters qui comprennent du carbone d'origine renouvelable.

En effet, à la différence des matériaux issus de matières fossiles, les matériaux composés en partie de matières premières renouvelables contiennent du ¹⁴C . Tous les échantillons de carbone tirés d'organismes vivants (animaux ou végétaux) sont en fait un mélange de 3 isotopes : ¹²C (représentant ~ 98,892 %), ¹³C (~ 1,108 %) et ¹⁴C (traces: 1,2.10⁻¹⁰ %). Le rapport ¹⁴C /¹²C des tissus vivants est identique à celui de l'atmosphère. Dans l'environnement, le ¹⁴C existe sous deux formes prépondérantes : sous forme minérale c'est-à-dire de gaz carbonique (CO₂) et sous forme organique c'est à dire de carbone intégré dans des molécules organiques.

Dans un organisme vivant, le rapport ¹⁴C /¹²C est maintenu constant par le métabolisme car le carbone est continuellement échangé avec l'environnement. La proportion de ¹⁴C étant constante dans l'atmosphère, il en est de même dans l'organisme, tant qu'il est vivant, puisqu'il absorbe ce ¹⁴C comme il absorbe le ¹²C. Le rapport moyen de ¹⁴C /¹²C est égal à 1,2x10⁻¹².

Le ¹²C est stable, c'est-à-dire que le nombre d'atomes de ¹²C dans un échantillon donné est constant au cours du temps. Le ¹⁴C, lui, est radioactif et chaque gramme de carbone d'être vivant contient suffisamment d'isotope ¹⁴C pour donner 13,6 désintégrations par minute.

La demi-vie (ou période T1/2), est la durée au bout de laquelle un nombre quelconque de noyaux radioactifs ou de particules instables d'une espèce donnée, est réduit de moitié par désintégration.. La demi-vie du ¹⁴C est de 5730 ans.

Compte tenu de cette demi-vie (T1/2) du ¹⁴C, on considère que la teneur en ¹⁴C est constante depuis l'extraction des matières premières végétales jusqu'à la fabrication de l'aminoacide ou aminoester, et même jusqu'à la fin de son utilisation.

Un aminoacide ou aminoester est en partie issu de matières premières renouvelables s'il contient au moins 20% en masse de C d'origine renouvelable, de préférence au moins 50 % en masse de C d'origine renouvelable Le demandeur considère qu'un aminoacide ou aminoester est issu de matières premières renouvelables et donc « bio » s'il contient entre 0,6.10⁻¹⁰ % et 1.2 10-¹⁰ % en masse de ¹⁴C.

A l'heure actuelle, il existe au moins deux techniques différentes pour la mesure de la teneur en ¹⁴C d'un échantillon :
- Par spectrométrie à scintillation liquide :
- Par spectrométrie de masse : l'échantillon est réduit en graphite ou en CO₂ gazeux, analysé dans un spectromètre de masse. Cette technique utilise un accélérateur et un spectromètre de masse pour séparer les ions ¹⁴C des ¹²C et donc déterminer le rapport des deux isotopes.

Toutes ces méthodes de mesure de la teneur en ¹⁴C des matériaux sont décrites précisément dans les normes ASTM D 6866 (notamment D6866-06) et dans les normes ASTMD 7026 (notamment 7026-04). »

La méthode de mesure préférentiellement utilisée dans le cas des aminoacides ou aminoesters de l'invention est la scintillation (Liquid Scintillation Counting LSC) décrite dans la norme ASTM D6866-06.

L'invention est illustrée par les exemples suivants.

### Exemple 1

Cet exemple illustre la première étape d'éthénolyse de l'acide palmitoléique Cette réaction est conduite à 30°C à la pression atmosphérique en présence d'un catalyseur à base de Ruthénium [RuCl₂(=CHPh)(IMesH₂)(PCy₃)] en utilisant un excès d'éthylène pour obtenir l'acide 9-décénoïque CH₂=CH-(CH₂)₇-COOH.. Les rendements sont déterminés par analyse chromatographique. Au terme de la réaction, 6 heures, on sépare l'α-oléfine en C8 par distillation sous vide.

### Exemple 2

Cet exemple illustre la deuxième étape (facultative) d'homométathèse de l'acide 9-décénoïque issu de la première étape en diacide de formule COOH-(CH₂)₇-CH=CH-(CH₂)₇-COOH.

Pour cette deuxième étape, on utilise le catalyseur complexe bispyridine ruthénium (8) décrit dans la publication de Chen-Xi Bai et al., Tetrahedron Letters, 46 (2005) 7225-7228. La réaction est effectuée dans le toluène, à une température de 50°C et pendant 12 heures sous une pression de 50 kPa en extrayant en cours de réaction l'éthylène formé. Le rendement est de 90 % molaire.

### Exemple 3

Cet exemple illustre la coupure oxydante de l'acide 9-décénoïque issu de l'exemple 1 par ozonolyse réductrice.
De l'ozone obtenue par un générateur d'ozone Welsbach T-408, est bullée dans 25 ml de pentane jusqu'à ce qu'une couleur bleue soit observée. La solution de pentane est maintenue à -70°C avec un bain d'acétone-carboglace. 20 mg d'ester méthylique, dissout dans 5 ml de pentane refroidis à 0 °C sont ajoutés à la solution d'ozone. L'excès d'ozone est ensuite éliminé et la couleur bleue disparaît. Après 5 minutes, le pentane est évaporé par un flux d'azote sec. Pendant cette étape la température de la solution est maintenue inférieure à 0°C. Après évaporation du pentane, 3 ml de méthanol refroidi à -70°C sont ajoutés au réacteur, tout en le réchauffant pour permettre la dissolution de l'ozonide. La solution claire est refroidie à -70°C, puis 2 ml de DMS sont ajoutés. Le réacteur est réchauffé de temps en temps pour permettre une dissolution correcte des précipités éventuellement formés. La solution est maintenue à -70°C pour 20 minutes et est ensuite réchauffée lentement. Elle est maintenue 15 minutes à 0°C avant d'être amenée à température ambiante. L'excès de DMS est évaporé sous flux d'azote sec et le méthanol est ensuite évaporé sous vide et l'on obtient l'acide 9-oxononanoïque avec un rendement de 50 %.molaire par rapport au théorique.

### Exemple 4

Cet exemple illustre la coupure oxydante du diacide C18 issu de l'exemple 2 par ozonolyse réductrice.

1 mg du diester de l'acide oléique : diméthyl -1,18 octadéc-9-énoate, est dissout dans 2 ml de pentane saturé d'ozone et prérefroidis à -70°C. Le pentane est évaporé sous flux d'azote et 1 ml de DMS est ajouté à l'ozonide obtenu. Après 30 minutes, l'excès de DMS est évaporé sous flux d'azote. Le produit est dissout dans une petite quantité d'éther et est analysé.
Le rendement en ester 9-oxononanoate de méthyle est de 82 % molaire.

### Exemple 5

Cet exemple illustre les étapes 2 et 3 du procédé (sans homométathèse) et notamment et notamment l'amino-réduction de la fonction aldéhyde de l'acide ω-oxononanoïque.

Dans un ballon de 2 litres surmonté d'un réfrigérant, on chauffe à reflux pendant 6 heures 530 g d'acide 9-décénoïque, 1600 ml de méthanol et 35 ml d'acide sulfurique concentré. Au refroidissement, le mélange se sépare en deux parties : le décénoate de méthyle et le méthanol en excès. La majeure partie de l'alcool est éliminé à la trompe à eau, puis le décénoate qui surnage est décanté de l'alcool restant. On ajoute de l'eau à cet alcool et on extrait à l'éther. Les solutions éthérées et le décénoate de méthyle sont mélangés, lavés à l'eau, puis avec une solution de bicarbonate de Na et enfin à l'eau. On sèche sur sulfate de sodium, on évapore l'éther, on chasse l'alcool restant à la trompe à eau et on distille à la pompe à palettes. On recueille 526 g de décénoate très légèrement jaune.

De l'oxygène ozoné (ozoniseur T23 de Trailigaz-Welsbach) passe à un débit de 100 l/h à travers une solution de 100 g de décénoéate dans 100 cc d'acide acétique glacial. L'ozonisation dure 7 heures et est suivie par la coloration d'une solution d'iodure de potassium à 2 %. Le courant d'ozone est arrêté lorsque l'iode précipite et qu'une solution d'iodure de potassium mise en dérivation sur le circuit de sortie des gaz jaunit instantanément. Si on arrête l'ozone dès l'apparition de la couleur rouge dans la première solution, l'ozonization est incomplète, comme le prouve la décoloration d'une solution de brome dans l'acide acétique glacial par une goutte de la solution à ozoniser.

L'ozonisation est exothermique et la solution à ozoniser doit être refroidie par de l'eau. Lorsque la réaction est terminée, un courant d'oxygène balaye pendant une demi-heure tout l'appareillage et chasse, entre autres, l'ozone dissous dans la solution acétique. Cette solution est diluée par 400 ml d'éther et versée dans un ballon tricol de 2 litres muni d'un agitateur et d'un réfrigérant. On agite et on verse d'un seul coup 10 g de zinc en poudre avec 0,5 ml d'eau puis 90 g de zinc par petites portions et enfin 10 ml d'eau goutte à goutte.

La vitesse à laquelle la poudre de zinc et l'eau doivent être ajoutés dépend de la vigueur de la décomposition et le refroidissement peut parfois être nécessaire. Après l'addition du zinc et de l'eau, le mélange est reflué jusqu'à ce qu'il ne donne plus de coloration bleue quand une goutte est agitée avec une solution d'iodure de potassium et de thiodène.

L'acétate de zinc est alors filtré sur Büchner et lavé soigneusement à l'éther. Le filtrat et les solutions de lavage sont agitées deux fois avec 500 ml d'eau, une fois avec 200 ml et d'une solution de carbonate de sodium à 10 % et enfin avec de l'eau. On sèche sur sulfate de sodium, chasse l'éther, distille à la trompe à eau, puis à la pompe à palettes.

On recueille ainsi 4 fractions : une fraction de tête contenant les aldéhydes légers, une fraction intermédiaire, une fraction riche en aldéhyde-ester et une fraction de queue.
Le rendement en aldéhyde ester est de 75 % molaire.

Dans un autoclave en acier inoxydable de 500 ml, on verse 50 g d'aldéhyde ester, 50 ml d'ammoniac liquide, 125 ml d'alcool et 6 g de Nickel de Raney.
L'hydrogène est introduit à une pression de 100 à 150 atmosphères et l'autoclave est chauffé à 100 - 110 °C pendant 4 heures. Au refroidissement l'hydrogène et l'ammoniac sont chassés, le contenu est siphonné et l'autoclave est rincé avec l'alcool. Le contenu de l'autoclave et l'alcool de lavage sont rassemblés, essorés sur Büchner et placés dans un appareil à distiller sous vide en présence d'azote. L'alcool et l'ammoniac sont chassés à la trompe à eau puis à la pompe à palettes. L'aminoester brut, coloré, est placé dans une ampoule à brome en vue de sa distillation dans l'appareil décrit.
L'aminoester distillé (38 g) est légèrement coloré. Le rendement est de 76 % molaire.

L'aminoester peut éventuellement directement polymérisé en polyamide-9, par chauffage sous vide pour récupérer le méthanol produit.

On peut aussi polymériser l'aminoacide. Pour cela on procède à une hydrolyse de l'amino ester. L'amino-9-nonanoate de méthyle obtenu à partir de 28 g d'aldéhyde ester est placé dans une ampoule à brome pour tomber goutte à goutte dans un ballon tricol de 2 litres surmonté d'une longue colonne à distiller et contenant un litre d'eau bouillante. Le reflux est réglé de façon à distiller le méthanol formé, ce qui permet de suivre la réaction, l'hydrolyse dure 4 à 5 heures pour l'ester méthylique. Lorsque la réaction est terminée, on filtre à chaud et évapore l'eau. On obtient un produit difficile à sécher au dessiccateur alors qu'en lavant le produit humide à l'acétone et en le séchant au dessiccateur, on recueille 20 g d'acide aminé brut incolore.

## Revendications

1. Procédé de synthèse d'aminoacides ou d'aminoesters de formule générale NH₂-(CH₂)₈-COOR , R étant soit H, soit un radical alkyle comportant de 1 à 4 atomes de carbone, à partir d'acides ou d'esters gras naturels insaturés à longue chaîne répondant à la formule R₁-CH=CH-(CH₂)₇-COOR dans laquelle R₁ représente soit H, soit un radical hydrocarboné comprenant de 1 à 11 atomes de carbone, comportant de 0 à 2 insaturations oléfiniques et le cas échéant une fonction hydroxyle, consistant, dans une première étape à soumettre ledit acide ou ester gras insaturé à une réaction de métathèse catalytique croisée avec l'éthylène pour former des acides ou esters ω-insaturés dont au moins 50% molaires répondent à la formule CH₂=CH-(CH₂)₇-COOR, puis dans une deuxième étape à soumettre l'acide ou ester de formule CH₂=CH-(CH₂)₇-COOR à une réaction de coupure oxydante pour former un aldéhyde-acide/ester de formule CHO-(CH₂)₇-COOR , puis enfin à transformer le produit résultant par amination réductrice en ω-amino-acide/ester de formule NH₂-(CH₂)₈-COOR.

2. Procédé selon la revendication 1 **caractérisé en ce que** la coupure oxydante est réalisée par ozonolyse réductrice.

3. Procédé selon la revendication 2 **caractérisé en ce que** la fonction réduction de l'ozonolyse réductrice est assurée par du zinc métal.

4. Procédé selon la revendication 2 **caractérisé en ce que** la fonction réduction de l'ozonolyse réductrice est réalisée au moyen de diméthylesulfure.

5. Procédé selon l'une des revendications 1 à 4 **caractérisé en ce que** au terme de la première étape, on sépare l'α-oléfine du milieu pour soumettre lors d'une deuxième étape l'acide/ester insaturé de formule CH₂=CH-(CH₂)₇-COOR à une homométathèse pour former le diacide(diester) de formule ROOC-(CH₂)₇-CH=CH-(CH₂)₇-COOR, puis, dans une troisième étape on soumet le diacide(diester) COOR-(CH₂)₇-CH=CH-(CH₂)₇-COOR à une réaction de coupure oxydante pour former un aldéhyde-acide de formule CHO-(CH₂)₇-COOR , puis enfin, dans une quatrième étape à transformer le produit résultant par une amination réductrice en ω-amino-acide/ester de formule NH₂-(CH₂)₈-COOR

## Claims

1. Process for the synthesis of amino acids or amino esters of general formula NH₂-(CH₂)₈-COOR, R being either H or an alkyl radical comprising from 1 to 4 carbon atoms, from long-chain natural unsaturated fatty acids or esters corresponding to the formula R₁CH=CH-(CH₂)₇-COOR in which R₁ represents either H or a hydrocarbon radical comprising from 1 to 11 carbon atoms, comprising from 0 to 2 olefinic unsaturations and, if appropriate, comprising one hydroxyl functional group, which consists, in a first stage, in subjecting said unsaturated fatty acid or ester to a catalytic cross metathesis reaction with ethylene to form ω-unsaturated acids or esters, at least 50 mol% of which correspond to the formula CH₂=CH-(CH₂)₇-COOR, then, in a second stage, in subjecting the acid or ester of formula CH₂=CH-(CH₂)₇-COOR to an oxidative cleavage reaction to form an aldehyde acid/ester of formula CHO-(CH₂)₇-COOR and then, finally, in converting the resulting product by reductive amination to give an ω-amino acid/ester of formula NH₂-(CH₂)₈-COOR.

2. Process according to Claim 1, **characterized in that** the oxidative cleavage is carried out by reductive ozonolysis.

3. Process according to Claim 2, **characterized in that** the reduction function of the reductive ozonolysis is provided by zinc metal.

4. Process according to Claim 2, **characterized in that** the reduction function of the reductive ozonolysis is carried out using dimethyl sulphide.

5. Process according to one of Claims 1 to 4, **characterized in that**, on completion of the first stage, the α-olefin is separated from the medium in order to subject the unsaturated acid/ester of formula CH₂=CH-(CH₂)₇-COOR, during a second stage, to a homometathesis in order to form the diacid (diester) of formula ROOC-(CH₂)₇-CH=CH-(CH₂)₇-COOR, then, in a third stage, the diacid (diester) COOR-(CH₂)₇-CH=CH-(CH₂)₇-COOR is subjected to an oxidative cleavage reaction to form an aldehyde acid of formula CHO-(CH₂)₇-COOR and then, finally, in a fourth stage, the resulting product is converted by reductive amination to give an ω-amino acid/ester of formula NH₂-(CH₂)₈-COOR.

## Patentansprüche

1. Verfahren zur Synthese von Aminosäuren oder Aminoestern der allgemeinen Formel NH₂-(CH₂)₈-COOR, wobei R für H oder einen Alkylrest mit 1 bis 4 Kohlenstoffatomen steht, aus langkettigen ungesättigten natürlichen Fettsäuren oder Fettestern der Formel R₁-CH=CH-(CH₂)₇-COOR, wobei R₁ für H oder einen Kohlenwasserstoffrest mit 1 bis 11 Kohlenstoffatomen und 0 bis 2 olefinischen Ungesättigtheiten und gegebenenfalls einer Hydroxylgruppe steht, bei dem man in einem ersten Schritt die ungesättigte Festtsäure bzw. den ungesättigten Fettester einer katalytischen Kreuzmetathesereaktion mit Ethylen zur Bildung von ω-ungesättigten Säuren bzw. Estern, von denen mindestens 50 Mol-% der Formel CH₂=CH-(CH₂)₇-COOR entsprechen, dann in einem zweiten Schritt die Säure bzw. den Ester der Formel CH₂=CH-(CH₂)₇-COOR einer oxidativen Spaltungsreaktion zur Bildung einer Aldehydsäure bzw. eines Aldehydesters der Formel CHO-(CH₂)₇-COOR unterwirft und schließlich das erhaltene Produkt durch reduktive Aminierung in eine ω-Aminosäure bzw. einen ω-Aminoester der Formel NH₂-(CH₂)₈-COOR umwandelt.

2. Verfahren nach Anspruch 1, **dadurch gekennzeichnet, dass** man die oxidative Spaltung durch reduktive Ozonolyse durchführt.

3. Verfahren nach Anspruch 2, **dadurch gekennzeichnet, dass** die Reduktionsfunktion der reduzierenden Ozonolyse durch Zinkmetall gewährleistet wird.

4. Verfahren nach Anspruch 2, **dadurch gekennzeichnet, dass** die Reduktionsfunktion der reduzierenden Ozonolyse mittels Dimethylsulfid gewährleistet wird.

5. Verfahren nach einem der Ansprüche 1 bis 4, **dadurch gekennzeichnet, dass** man am Ende des ersten Schritts das α-Olefin von dem Medium abtrennt, um in einem zweiten Schritt die ungesättigte Säure/den ungesättigten Ester der Formel CH₂=CH-(CH₂)₇-COOR einer Homometathesereaktion zur Bildung der Disäure (des Diesters) der Formel ROOC-(CH₂)₇-CH=CH-(CH₂)₇-COOR zu unterwerfen, und in einem dritten Schritt die Disäure (den Diester) COOR-(CH₂)₇-CH=CH-(CH₂)₇-COOR einer oxidativen Spaltungsreaktion zur Bildung einer Aldehydsäure der Formel CHO-(CH₂)₇-COOR unterwirft und schließlich in einem vierten Schritt das erhaltene Produkt durch reduktive Aminierung in eine ω-Aminosäure bzw. einen ω-Aminoester der Formel NH₂-(CH₂)₈-COOR umwandelt.
